# EUROPEAN PATENT APPLICATION

(11) **EP 1 548 434 A1**
(43) Date of publication of application: **29.06.2005**
(21) Application number: 03784625.0
(22) Date of filing: 08.08.2003
(51) Int. Cl.: G01N 33/50, G01N 33/15, C12Q 1/02

(54) **METHOD OF SCREENING DRUG**

(30) Priority: 08.08.2002 JP 2002231999
(71) Applicant: Japan Science and Technology Agency, Kawaguchi-shi, Saitama 332-0012 (JP); OSAKA BIOSCIENCE INSTITUTE, Suita-shi, Osaka-Fu 565-0874 (JP)
(72) Inventor: KAKIZUKA, Akira Osaka Bioscience Institute, Suita-shi, Osaka 565-0874 (JP); KAMEI, Yasutomi Osaka Bioscience Institute, Suita-shi, Osaka 565-0874 (JP); OHIZUMI, Hiroshi Osaka Bioscience Institute, Suita-shi, Osaka 565-0874 (JP)
(74) Representative: Grant, Anne Rosemary
(86) International application number: PCT/JP2003/010163
(87) International publication number: WO 2004/015415

(57) **Abstract**

A method of screening a substance which serves as the active ingredient in a drug for obesity and/or diabetes is provided. This method comprises treating cells or an animal individual with a candidate substance and specifying a candidate substance fulfilling one or more of the following requirements as the target active ingredient substance: (a) increasing the expression level of a ligand factor ERRL1 for a nuclear receptor ERR; (b) increasing the transcriptional activity of the nuclear receptor ERR; (c) promoting the binding of ERRL1 to ERR; and (d) increasing the expression level of an MCAD gene product.

SEQUENCE LISTING

<110> Japan science and Technology Corporation

<120> A method of drug screening

<130> 03-F-039PCT

<140> PCT/JP2003/010163
<141> 2003-08-08

<150> JP 2002-231999
<151> 2002-08-08

<160> 2

<170> PatentIn Ver. 2.1

<210> 1
<211> 3345
<212> DNA
<213> Homo sapiens

<220>
<221> CDS
<222> (32)..(3076)

<400> 1
ctccgccgca cgctgcagcc gcggctggaa g atg gcg ggg aac gac tgc ggc 52
Met Ala Gly Asn Asp Cys Gly
1               5

gcg ctg ctg gat gaa gag ctc tcg tcc ttc ttc ctc aac tat ctc tct 100
Ala Leu Leu Asp Glu Glu Leu Ser Ser Phe Phe Leu Asn Tyr Leu Ser
        10                  15                  20

gac acg cag ggt ggg gac tct gga gag gaa cag ctg tgt gct gac ttg 148
Asp Thr Gln Gly Gly Asp Ser Gly Glu Glu Gln Leu Cys Ala Asp Leu
    25                  30                  35

cca gag ctt gac ctc tcc cag ctg gac gcc agt gac ttt gac tca gcc 196
Pro Glu Leu Asp Leu Ser Gln Leu Asp Ala Ser Asp Phe Asp Ser Ala
40                  45                  50                  55

acg tgc ttt ggg gag ctg cag tgg tgc ccg gag acc tca gag aca gag 244
Thr Cys Phe Gly Glu Leu Gln Trp Cys Pro Glu Thr Ser Glu Thr Glu
                60                  65                  70

ccc agc cag tac agc ccc gat gac tcc gag ctc ttc cag att gac agt 292
Pro Ser Gln Tyr Ser Pro Asp Asp Ser Glu Leu Phe Gln Ile Asp Ser
            75                  80                  85

gag aat gaa gct ctc ttg gct gcg ctt acg aag acc ctg gat gac atc 340
Glu Asn Glu Ala Leu Leu Ala Ala Leu Thr Lys Thr Leu Asp Asp Ile
        90                  95                  100

ccc gaa gac gat gtg ggg ctg gct gcc ttc cca gaa ctg gat gaa ggc 388
Pro Glu Asp Asp Val Gly Leu Ala Ala Phe Pro Glu Leu Asp Glu Gly
    105                 110                 115

gac aca cca tcc tgc acc cca gcc tca cct gcc ccc tta tct gca ccc 436
Asp Thr Pro Ser Cys Thr Pro Ala Ser Pro Ala Pro Leu Ser Ala Pro
120                 125                 130                 135

ccc agc ccc acc ctg gag agg ctt ctg tcc cca gcg tct gac gtg gac 484
Pro Ser Pro Thr Leu Glu Arg Leu Leu Ser Pro Ala Ser Asp Val Asp
                140                 145                 150

gag ctt tca ctg cta cag aag ctc ctc ctg gcc aca tcc tcc cca aca 532
Glu Leu Ser Leu Leu Gln Lys Leu Leu Leu Ala Thr Ser Ser Pro Thr
            155                 160                 165

gca agc tct gac gct ctg aag gac ggg gcc acc tgg tcc cag acc agc 580
Ala Ser Ser Asp Ala Leu Lys Asp Gly Ala Thr Trp Ser Gln Thr Ser
        170                 175                 180

ctc agt tcc aga agt cag cgg cct tgt gtc aag gtg gat ggc acc cag 628
Leu Ser Ser Arg Ser Gln Arg Pro Cys Val Lys Val Asp Gly Thr Gln
    185                 190                 195

gat aag aag acc ccc aca ctg cgg gct cag agc cgg cct tgt acg gaa 676
Asp Lys Lys Thr Pro Thr Leu Arg Ala Gln Ser Arg Pro Cys Thr Glu
200                 205                 210                 215

ctg cat aag cac ctc act tcg gtg ctg ccc tgt ccc aga gtg aaa gcc 724
Leu His Lys His Leu Thr Ser Val Leu Pro Cys Pro Arg Val Lys Ala

                220                 225                 230

tgc tcc cca act ccg cac ccg agc cct cgg ctc ctc tcc aaa gag gag 772
Cys Ser Pro Thr Pro His Pro Ser Pro Arg Leu Leu Ser Lys Glu Glu
            235                 240                 245

gag gag gag gtg ggg gag gat tgc cca agc cct tgg ctg act cca gcc 820
Glu Glu Glu Val Gly Glu Asp Cys Pro Ser Pro Trp Leu Thr Pro Ala
        250                 255                 260

tcg ccc caa gac tcc cta gca cag gac acg gcc agc ccc gac agt gcc 868
Ser Pro Gln Asp Ser Leu Ala Gln Asp Thr Ala Ser Pro Asp Ser Ala
    265                 270                 275

cag cct ccc gag gag gat gtg agg gcc atg gta cag ctc att cgc tac 916
Gln Pro Pro Glu Glu Asp Val Arg Ala Met Val Gln Leu Ile Arg Tyr
280                 285                 290                 295

atg cat acc tac tgc ctg cct cag agg aag ctg ccc caa cgg gcc cca 964
Met His Thr Tyr Cys Leu Pro Gln Arg Lys Leu Pro Gln Arg Ala Pro
                300                 305                 310

gag cca atc ccc cag gcc tgc agc agc ctc tcc agg cag gtt caa ccc 1012
Glu Pro Ile Pro Gln Ala Cys Ser Ser Leu Ser Arg Gln Val Gln Pro
            315                 320                 325

cga tcc cgg cat ccc ccc aaa gcc ttc tgg act gag ttc tct atc cta 1060
Arg Ser Arg His Pro Pro Lys Ala Phe Trp Thr Glu Phe Ser Ile Leu
        330                 335                 340

agg gaa ctt ctg gcc caa gat atc ctc tgt gat gtt agc aag ccc tac 1108
Arg Glu Leu Leu Ala Gln Asp Ile Leu Cys Asp Val Ser Lys Pro Tyr
    345                 350                 355

cgc ctg gcc ata cct gtc tat gct tcc ctc aca cct cag tcc agg ccc 1156
Arg Leu Ala Ile Pro Val Tyr Ala Ser Leu Thr Pro Gln Ser Arg Pro
360                 365                 370                 375

agg ccc ccc aag gac agt cag gcc tcc cct gcc cac tct gcc atg gca 1204
Arg Pro Pro Lys Asp Ser Gln Ala Ser Pro Ala His Ser Ala Met Ala
                380                 385                 390

gaa gag gtg aga atc act gct tcc ccc aag agc acc ggg cct aga ccc 1252

Glu Glu Val Arg Ile Thr Ala Ser Pro Lys Ser Thr Gly Pro Arg Pro
            395                 400                 405

agc ctg cgt cct ctg agg ctg gag gtg aaa cgg gat gtt aac aag cct 1300
Ser Leu Arg Pro Leu Arg Leu Glu Val Lys Arg Asp Val Asn Lys Pro
        410                 415                 420

aca agg caa aag cgg gag gaa gat gag gag gag gag gag gaa gaa gaa 1348
Thr Arg Gln Lys Arg Glu Glu Asp Glu Glu Glu Glu Glu Glu Glu Glu
    425                 430                 435

gaa gag gaa gaa gaa aaa gaa gag gaa gaa gag gag tgg ggc agg aag 1396
Glu Glu Glu Glu Glu Lys Glu Glu Glu Glu Glu Glu Trp Gly Arg Lys
440                 445                 450                 455

aga cca ggt cgt ggc ctg cca tgg acc aaa cta ggg agg aag atg gac 1444
Arg Pro Gly Arg Gly Leu Pro Trp Thr Lys Leu Gly Arg Lys Met Asp
                460                 465                 470

agc tcc gtg tgc ccc gtg cgg cgc tcc agg aga ctg aat cca gag ctg 1492
Ser Ser Val Cys Pro Val Arg Arg Ser Arg Arg Leu Asn Pro Glu Leu
            475                 480                 485

ggt ccc tgg ctg aca ttc act gat gag ccc tta ggt gct ctg ccc tcg 1540
Gly Pro Trp Leu Thr Phe Thr Asp Glu Pro Leu Gly Ala Leu Pro Ser
        490                 495                 500

atg tgc ctg gat aca gag acc cac aac ctg gag gaa gac ctg ggc agc 1588
Met Cys Leu Asp Thr Glu Thr His Asn Leu Glu Glu Asp Leu Gly Ser
    505                 510                 515

ctc aca gac agt agt caa ggc cgg cag ctc ccc cag gga tcc cag atc 1636
Leu Thr Asp Ser Ser Gln Gly Arg Gln Leu Pro Gln Gly Ser Gln Ile
520                 525                 530                 535

ccc gcc ctg gaa agc ccc tgt gag agt ggg tgc gga gac aca gat gaa 1684
Pro Ala Leu Glu Ser Pro Cys Glu Ser Gly Cys Gly Asp Thr Asp Glu
                540                 545                 550

gat cca agc tgc cca cag ccc act tcc aga gac tcc tcc agg tgc ctc 1732
Asp Pro Ser Cys Pro Gln Pro Thr Ser Arg Asp Ser Ser Arg Cys Leu
            555                 560                 565

atg ctg gcc ttg tca caa agc gac tct ctt ggc aag aag agc ttt gag 1780
Met Leu Ala Leu Ser Gln Ser Asp Ser Leu Gly Lys Lys Ser Phe Glu
        570                 575                 580

gag tcc ctg acg gtg gag ctt tgc ggc acg gca gga ctc acg cca ccc 1828
Glu Ser Leu Thr Val Glu Leu Cys Gly Thr Ala Gly Leu Thr Pro Pro
    585                 590                 595

acc aca cct cca tac aag cca atg gag gag gac ccc ttc aag cca gac 1876
Thr Thr Pro Pro Tyr Lys Pro Met Glu Glu Asp Pro Phe Lys Pro Asp
600                 605                 610                 615

acc aag ctc agc cca ggc caa gac aca gct ccc agc ctt ccc tcc ccc 1924
Thr Lys Leu Ser Pro Gly Gln Asp Thr Ala Pro Ser Leu Pro Ser Pro
                620                 625                 630

gag gct ctt ccg ctc aca gcc acc cca gga gct tcc cac aag ctg ccc 1972
Glu Ala Leu Pro Leu Thr Ala Thr Pro Gly Ala Ser His Lys Leu Pro
            635                 640                 645

aag agg cac cca gag cga agc gag ctc ctg tcc cat ttg cag cat gcc 2020
Lys Arg His Pro Glu Arg Ser Glu Leu Leu Ser His Leu Gln His Ala
        650                 655                 660

aca acc caa cca gtc tca cag gct ggc cag aag cgc ccc ttc tcc tgc 2068
Thr Thr Gln Pro Val Ser Gln Ala Gly Gln Lys Arg Pro Phe Ser Cys
    665                 670                 675

tcc ttt gga gac cac gac tac tgc cag gtg ctc agg cca gag gct gcc 2116
Ser Phe Gly Asp His Asp Tyr Cys Gln Val Leu Arg Pro Glu Ala Ala
680                 685                 690                 695

ctg cag agg aag gtg ctg cgg tcc tgg gag cca atc ggg gtc cac ctt 2164
Leu Gln Arg Lys Val Leu Arg Ser Trp Glu Pro Ile Gly Val His Leu
                700                 705                 710

gaa gac ttg gcc cag cag ggt gcc cct ctg cca acg gaa aca aag gcc 2212
Glu Asp Leu Ala Gln Gln Gly Ala Pro Leu Pro Thr Glu Thr Lys Ala
            715                 720                 725

cct agg agg gag gca aac cag aac tgt gac cct acc cac aag gac agc 2260
Pro Arg Arg Glu Ala Asn Gln Asn Cys Asp Pro Thr His Lys Asp Ser
        730                 735                 740

atg cag cta aga gac cat gag atc cgt gcc agt ctc aca aag cac ttt 2308
Met Gln Leu Arg Asp His Glu Ile Arg Ala Ser Leu Thr Lys His Phe
    745                 750                 755

ggg ctg ctg gag act gct ctg gaa ggt gaa gac ctg gcg tcc tgt aaa 2356
Gly Leu Leu Glu Thr Ala Leu Glu Gly Glu Asp Leu Ala Ser Cys Lys
760                 765                 770                 775

agc ccg gag tat gac acc gta ttt gag gac agc agc agc agc agt ggc 2404
Ser Pro Glu Tyr Asp Thr Val Phe Glu Asp Ser Ser Ser Ser Ser Gly
                780                 785                 790

gag agt agc ttc ctg ctt gag gag gag gag gaa gag gag gag gga ggg 2452
Glu Ser Ser Phe Leu Leu Glu Glu Glu Glu Glu Glu Glu Glu Gly Gly
            795                 800                 805

gaa gag gac gat gaa gga gag gac tca ggg gtc agc cct ccc tgc tct 2500
Glu Glu Asp Asp Glu Gly Glu Asp Ser Gly Val Ser Pro Pro Cys Ser
        810                 815                 820

gat cac tgc ccc tac cag agc cca ccc agt aag gcc agt cgg cag ctc 2548
Asp His Cys Pro Tyr Gln Ser Pro Pro Ser Lys Ala Ser Arg Gln Leu
    825                 830                 835

tgc tcc cga agc cgc tcc agt tcc ggc tcc tcg tcc tgc agc tcc tgg 2596
Cys Ser Arg Ser Arg Ser Ser Ser Gly Ser Ser Ser Cys Ser Ser Trp
840                 845                 850                 855

tca cca gcc acc cgg aag aac ttc aga cgt gag agc aga ggg ccc tgt 2644
Ser Pro Ala Thr Arg Lys Asn Phe Arg Arg Glu Ser Arg Gly Pro Cys
                860                 865                 870

tca gat gga acc cca agc gtc cgg cat gcc agg aag cgg cgg gaa aag 2692
Ser Asp Gly Thr Pro Ser Val Arg His Ala Arg Lys Arg Arg Glu Lys
            875                 880                 885

gcc atc ggt gaa ggc cgt gtg gta tac att cga aat ctc tcc agt gac 2740
Ala Ile Gly Glu Gly Arg Val Val Tyr Ile Arg Asn Leu Ser Ser Asp
        890                 895                 900

atg agc tct cgg gaa cta aag aag cgc ttt gag gtg ttc ggt gag att 2788
Met Ser Ser Arg Glu Leu Lys Lys Arg Phe Glu Val Phe Gly Glu Ile

905                 910                 915

gta gag tgc cag gtg ctg acg aga agt aaa aga ggc cag aag cac ggt    2836
Val Glu Cys Gln Val Leu Thr Arg Ser Lys Arg Gly Gln Lys His Gly
920                 925                 930                 935

ttt atc acc ttc cgg tgt tca gag cac gct gcc ctg tcc gtg agg aac    2884
Phe Ile Thr Phe Arg Cys Ser Glu His Ala Ala Leu Ser Val Arg Asn
                940                 945                 950

ggc gcc acc ctg aga aag cgc aat gag ccc tcc ttc cac ctg agc tat    2932
Gly Ala Thr Leu Arg Lys Arg Asn Glu Pro Ser Phe His Leu Ser Tyr
            955                 960                 965

gga ggg ctc cgg cac ttc cgt tgg ccc aga tac act gac tat gat ccc    2980
Gly Gly Leu Arg His Phe Arg Trp Pro Arg Tyr Thr Asp Tyr Asp Pro
        970                 975                 980

aca tct gag gag tcc ctt ccc tca tct ggg aaa agc aag tac gaa gcc    3028
Thr Ser Glu Glu Ser Leu Pro Ser Ser Gly Lys Ser Lys Tyr Glu Ala
    985                 990                 995

atg gat ttt gac agc tta ctg aaa gag gcc cag cag agc ctg cat tga    3076
Met Asp Phe Asp Ser Leu Leu Lys Glu Ala Gln Gln Ser Leu His
1000                1005                1010                1015

tatcagcctt aaccttcgag gaatacctca atacctcaga caaggccctt ccaatatgtt 3136

tacgttttca aagaaaagag tatatgagaa ggagagcgag cgagcgagcg agcgagcgag 3196

tgagcgtgag agatcacaca ggagagagaa agacttgaat ctgctgtcgt ttcctttaaa 3256

aaaaaaaaaa aaaaaactcg acggccaagt cggcctccct ttagtgaggg ttaatttgtg 3316

atcccgggtg gcatccctgt gacccctcc                                   3345

<210> 2
<211> 1014
<212> PRT
<213> Homo sapiens

<400> 2

Met Ala Gly Asn Asp Cys Gly Ala Leu Leu Asp Glu Glu Leu Ser Ser
1               5                   10                  15
Phe Phe Leu Asn Tyr Leu Ser Asp Thr Gln Gly Gly Asp Ser Gly Glu
            20                  25                  30
Glu Gln Leu Cys Ala Asp Leu Pro Glu Leu Asp Leu Ser Gln Leu Asp
        35                  40                  45
Ala Ser Asp Phe Asp Ser Ala Thr Cys Phe Gly Glu Leu Gln Trp Cys
    50                  55                  60
Pro Glu Thr Ser Glu Thr Glu Pro Ser Gln Tyr Ser Pro Asp Asp Ser
65                  70                  75                  80
Glu Leu Phe Gln Ile Asp Ser Glu Asn Glu Ala Leu Leu Ala Ala Leu
                85                  90                  95
Thr Lys Thr Leu Asp Asp Ile Pro Glu Asp Asp Val Gly Leu Ala Ala
            100                 105                 110
Phe Pro Glu Leu Asp Glu Gly Asp Thr Pro Ser Cys Thr Pro Ala Ser
        115                 120                 125
Pro Ala Pro Leu Ser Ala Pro Pro Ser Pro Thr Leu Glu Arg Leu Leu
    130                 135                 140
Ser Pro Ala Ser Asp Val Asp Glu Leu Ser Leu Leu Gln Lys Leu Leu
145                 150                 155                 160
Leu Ala Thr Ser Ser Pro Thr Ala Ser Ser Asp Ala Leu Lys Asp Gly
                165                 170                 175
Ala Thr Trp Ser Gln Thr Ser Leu Ser Ser Arg Ser Gln Arg Pro Cys
            180                 185                 190
Val Lys Val Asp Gly Thr Gln Asp Lys Lys Thr Pro Thr Leu Arg Ala
        195                 200                 205
Gln Ser Arg Pro Cys Thr Glu Leu His Lys His Leu Thr Ser Val Leu
    210                 215                 220
Pro Cys Pro Arg Val Lys Ala Cys Ser Pro Thr Pro His Pro Ser Pro
225                 230                 235                 240
Arg Leu Leu Ser Lys Glu Glu Glu Glu Glu Val Gly Glu Asp Cys Pro
                245                 250                 255
Ser Pro Trp Leu Thr Pro Ala Ser Pro Gln Asp Ser Leu Ala Gln Asp
            260                 265                 270
Thr Ala Ser Pro Asp Ser Ala Gln Pro Pro Glu Glu Asp Val Arg Ala
        275                 280                 285
Met Val Gln Leu Ile Arg Tyr Met His Thr Tyr Cys Leu Pro Gln Arg
    290                 295                 300
Lys Leu Pro Gln Arg Ala Pro Glu Pro Ile Pro Gln Ala Cys Ser Ser
305                 310                 315                 320
Leu Ser Arg Gln Val Gln Pro Arg Ser Arg His Pro Pro Lys Ala Phe
                325                 330                 335
Trp Thr Glu Phe Ser Ile Leu Arg Glu Leu Leu Ala Gln Asp Ile Leu

            340                 345                 350
Cys Asp Val Ser Lys Pro Tyr Arg Leu Ala Ile Pro Val Tyr Ala Ser
        355                 360                 365
Leu Thr Pro Gln Ser Arg Pro Arg Pro Pro Lys Asp Ser Gln Ala Ser
    370                 375                 380
Pro Ala His Ser Ala Met Ala Glu Glu Val Arg Ile Thr Ala Ser Pro
385                 390                 395                 400
Lys Ser Thr Gly Pro Arg Pro Ser Leu Arg Pro Leu Arg Leu Glu Val
                405                 410                 415
Lys Arg Asp Val Asn Lys Pro Thr Arg Gln Lys Arg Glu Glu Asp Glu
            420                 425                 430
Glu Glu Glu Glu Glu Glu Glu Glu Glu Glu Glu Glu Lys Glu Glu Glu
        435                 440                 445
Glu Glu Glu Trp Gly Arg Lys Arg Pro Gly Arg Gly Leu Pro Trp Thr
    450                 455                 460
Lys Leu Gly Arg Lys Met Asp Ser Ser Val Cys Pro Val Arg Arg Ser
465                 470                 475                 480
Arg Arg Leu Asn Pro Glu Leu Gly Pro Trp Leu Thr Phe Thr Asp Glu
                485                 490                 495
Pro Leu Gly Ala Leu Pro Ser Met Cys Leu Asp Thr Glu Thr His Asn
            500                 505                 510
Leu Glu Glu Asp Leu Gly Ser Leu Thr Asp Ser Ser Gln Gly Arg Gln
        515                 520                 525
Leu Pro Gln Gly Ser Gln Ile Pro Ala Leu Glu Ser Pro Cys Glu Ser
    530                 535                 540
Gly Cys Gly Asp Thr Asp Glu Asp Pro Ser Cys Pro Gln Pro Thr Ser
545                 550                 555                 560
Arg Asp Ser Ser Arg Cys Leu Met Leu Ala Leu Ser Gln Ser Asp Ser
                565                 570                 575
Leu Gly Lys Lys Ser Phe Glu Glu Ser Leu Thr Val Glu Leu Cys Gly
            580                 585                 590
Thr Ala Gly Leu Thr Pro Pro Thr Thr Pro Pro Tyr Lys Pro Met Glu
        595                 600                 605
Glu Asp Pro Phe Lys Pro Asp Thr Lys Leu Ser Pro Gly Gln Asp Thr
    610                 615                 620
Ala Pro Ser Leu Pro Ser Pro Glu Ala Leu Pro Leu Thr Ala Thr Pro
625                 630                 635                 640
Gly Ala Ser His Lys Leu Pro Lys Arg His Pro Glu Arg Ser Glu Leu
                645                 650                 655
Leu Ser His Leu Gln His Ala Thr Thr Gln Pro Val Ser Gln Ala Gly
            660                 665                 670
Gln Lys Arg Pro Phe Ser Cys Ser Phe Gly Asp His Asp Tyr Cys Gln
        675                 680                 685

Val Leu Arg Pro Glu Ala Ala Leu Gln Arg Lys Val Leu Arg Ser Trp
    690                 695                 700
Glu Pro Ile Gly Val His Leu Glu Asp Leu Ala Gln Gln Gly Ala Pro
705                 710                 715                 720
Leu Pro Thr Glu Thr Lys Ala Pro Arg Arg Glu Ala Asn Gln Asn Cys
                725                 730                 735
Asp Pro Thr His Lys Asp Ser Met Gln Leu Arg Asp His Glu Ile Arg
            740                 745                 750
Ala Ser Leu Thr Lys His Phe Gly Leu Leu Glu Thr Ala Leu Glu Gly
        755                 760                 765
Glu Asp Leu Ala Ser Cys Lys Ser Pro Glu Tyr Asp Thr Val Phe Glu
    770                 775                 780
Asp Ser Ser Ser Ser Ser Gly Glu Ser Ser Phe Leu Leu Glu Glu Glu
785                 790                 795                 800
Glu Glu Glu Glu Glu Gly Gly Glu Glu Asp Asp Glu Gly Glu Asp Ser
                805                 810                 815
Gly Val Ser Pro Pro Cys Ser Asp His Cys Pro Tyr Gln Ser Pro Pro
            820                 825                 830
Ser Lys Ala Ser Arg Gln Leu Cys Ser Arg Ser Arg Ser Ser Ser Gly
        835                 840                 845
Ser Ser Ser Cys Ser Ser Trp Ser Pro Ala Thr Arg Lys Asn Phe Arg
    850                 855                 860
Arg Glu Ser Arg Gly Pro Cys Ser Asp Gly Thr Pro Ser Val Arg His
865                 870                 875                 880
Ala Arg Lys Arg Arg Glu Lys Ala Ile Gly Glu Gly Arg Val Val Tyr
                885                 890                 895
Ile Arg Asn Leu Ser Ser Asp Met Ser Ser Arg Glu Leu Lys Lys Arg
            900                 905                 910
Phe Glu Val Phe Gly Glu Ile Val Glu Cys Gln Val Leu Thr Arg Ser
        915                 920                 925
Lys Arg Gly Gln Lys His Gly Phe Ile Thr Phe Arg Cys Ser Glu His
    930                 935                 940
Ala Ala Leu Ser Val Arg Asn Gly Ala Thr Leu Arg Lys Arg Asn Glu
945                 950                 955                 960
Pro Ser Phe His Leu Ser Tyr Gly Gly Leu Arg His Phe Arg Trp Pro
                965                 970                 975
Arg Tyr Thr Asp Tyr Asp Pro Thr Ser Glu Glu Ser Leu Pro Ser Ser
            980                 985                 990
Gly Lys Ser Lys Tyr Glu Ala Met Asp Phe Asp Ser Leu Leu Lys Glu
        995                 1000                1005
Ala Gln Gln Ser Leu His
    1010

## Description

### Technical Field

The invention of this application relates to a novel method of screening a drug for obesity and/or diabetes by using the expression and the activity of a ligand factor ERRL1 for a nuclear receptor ERR (estrogen receptor- related receptor) as an index.

### Background Art

The most typical molecular mechanism that underlies gene expression regulated by nuclear receptors starts from the binding of their ligands (e.g., small lipophilic molecules such as steroids, retinoic acid, thyroid hormone and vitamin D₃) (Mangelsdorf, D. J. et al., Cell 83: 835-839, 1995). Endogenous levels of such ligands are strictly regulated by means of multiple enzymatic reaction steps working toward their production and/or degradation (Honkakoski, P. & Negishi, M. Biochem. J. 347: 321-337, 2000). In addition, these ligands are collectively called lipophilic ligands or lipophilic hormones of the endocrine system. By changing ligand levels, the endocrine system contributes to the adaptation to changes in the in vitro or in vivo environment, namely to homeostasis (Mangelsdorf, D. J. et al., Cell 83: 835-839, 1995; Giguere, V. Endocr. Rev. 20: 689-725, 1999). This system appears advantageous for slow and long-term adaptations but disadvantageous for quick responses because of the complicated regulation of ligand production. On the other hand, genome analysis has predicted the existence of numerous nuclear receptor-like molecules. However, the lipophilic ligands specific to the molecules are currently hardly known, and these molecules are collectively called orphan receptors (Giguere, V. Endocr. Rev. 20: 689-725, 1999). The activation mechanisms of orphan receptors remain totally unknown. Estrogen receptor-related proteins 1 and 2 (ERR1 and -2) were the first identified orphan receptors (Giguere, V. et al., Nature 331: 91-94, 1988; Shigeta, H. et al., J. Mol. Endocrinol. 19: 299-309, 1997), and its third member (ERR3) has recently been isolated (Eudy, J. D. et al., Genomics 50: 382-384, 1998; Hong, H. et al., J. Biol. Chem. 274: 22618-22626, 1999). ERRs and estrogen receptors share structural similarity, however, ERRs do not respond to estrogen (Giguere, V. Endocr. Rev. 20: 689-725, 1999). On the other hand, ERR1 has been proposed to act as a key transcriptional regulator of the gene encoding medium-chain acyl CoA dehydrogenase (MCAD), namely a pivotal enzyme in mitochondrial fatty acid β-oxidation (Sladek, R. et al., Mol. Cell. Biol. 17: 5400-5409, 1997; Vega, R. B. & Kelly, D. P., J. Biol. Chem. 272: 31693-31699, 1997). These observations lead to the idea that ERR-mediated gene regulation may play important roles in the control of energy balance in the body by regulating fatty acid β-oxidation, which is generally induced by the physical exercises (Horowitz, J. F. & Klein, S., Am. J. Clin. Nutri. 72: 558S-563S, 2000). Therefore, daily performance of appropriate physical exercises is considered to be the simplest and most effective way to cope with obesity and diabetes (Baldwin, K. M., J. Appl. Physiol. 88: 332-336, 2000).

As described above, a nuclear orphan receptor, ERR serves as a regulator for MCAD gene expression, and it is considered that the resistance to obesity or diabetes can be maintained by controlling energy balance in the body by regulating fatty acid β-oxidation by this MCAD gene expression. However, the ligand molecule for the receptor ERR which is the origin of a series of such mechanisms has not been identified yet.

On the other hand, by researchers in the nuclear receptor field, it has been revealed that several classes of transcriptional cofactor proteins, such as SRC1/p160 family (Onate, S. A. et al., Science 270: 1354-1357, 1995), P/CAF (Blanco, J. C. G. et al., Genes Dev. 12: 1638-1651, 1998), and CBP/p300 (Chakravarti, D. et al., Nature, 383: 99-103, 1996; Kamei, Y. et al., Cell 85: 403-414, 1996) play key roles in ligand-dependent transcriptional activation of the nuclear receptors. These are called coactivators and are ubiquitously expressed. Further, their expression levels appear not to change during differentiation of cells or in response to changes in external and internal environments. More recently, a unique coactivator, termed PPARγ-coactivator-1 (PGC-1) was identified (Puigserver, P. et al., Cell, 92: 829-839, 1998). This coactivator distinguishes itself from other coactivators by means of its tissue-specific and regulated expression. In other words, PGC-1 is expressed in different levels in brown adipose tissue (BAT), skeletal muscle, heart, kidney, and brain and the expression is markedly up-regulated in BAT after acute exposure to cold stress (Puigserver, P. et al., Cell, 92: 829-839, 1998). PGC-1 expression is also up-regulated in the liver (Yoon, J. C. et al., Nature, 413: 131-138, 2001) and heart (Lehman, J. J. et al., J. Clin. Invest. 106: 847-856, 2000) under fasting conditions.

PPARγ is known to be the key regulator of adipogenesis (Tontonoz, P. et al., Cell 79, 1147-1156, 1994), and its expression is augmented during the differentiation of adipocytes. In addition, it has been revealed that thiazolidine (TZD), which has been developed as an improving agent for insulin resistance in diabetes, serves as the ligand for PPARγ (Lehmann, et al., J. Biol. Chem. 270: 12953-12956, 1995), and screening of novel drugs for diabetes using the transcriptional activation of PPARγ as an index has been widely carried out.

However, the level of PGC-1 mRNA, which is a coactivator of PPARγ, is at a very low level during adipocyte differentiation of 3T3-L1 cells. Therefore, it was speculated that a similar molecule to PGC-1, which may function during adipocyte differentiation might exist. The inventors of this application identified a novel binding factor, PGC2, to PPARγ, and has filed a patent application (JP-A-2002-058489) for the method of screening a ligand which regulates the transcriptional activity of PGC2-PPARγ complex (in particular, a ligand for decreasing activity of PGC2-PPARγ complex) as a means for developing a drug for insulin resistance diabetes.

### Disclosure of the invention

The inventors of this application searched for a ligand factor for a nuclear receptor ERR, a regulator for the expression of MCAD which is a resistance gene to obesity or diabetes. As a result, they found out that this factor is PGC2 which had been identified before as a ligand factor for PPARγ, and named this protein factor ERRL1 (abbreviation of ERR ligand 1).

The invention of this application is based on such a novel finding by the inventors, and makes it an object to provide a method of screening a drug targeting an endogenous pathway of anti-obesity and anti-diabetes related to the nuclear receptor ERR and its target gene MCAD, which is totally different from the action mechanism of PPARγ.

In order to solve the foregoing object, this invention provides a method of screening a substance which serves as the active ingredient in a drug for obesity and/or diabetes, which comprises treating cells or an animal with a candidate substance and specifying a candidate substance fulfilling one or more of the following requirements as the target active ingredient substance:
(a) increasing the expression level of a ligand factor ERRL1 for a nuclear receptor ERR;
(b) increasing the transcriptional activity of the nuclear receptor ERR;
(c) promoting the binding of ERRL1 to ERR; and
(d) increasing the expression level of an MCAD gene product.

In addition, this invention provides a drug for obesity and/or diabetes comprising as the active ingredient one or more substances specified by the foregoing screening method.

Further, this invention provides a transgenic non-human animal having a purified polynucleotide encoding a ligand factor ERRL1 for a nuclear receptor ERR in its genomic DNA and overexpressing the ligand factor ERRL1.

In this invention, 'polynucleotide' means a molecule in which phosphate esters of nucleosides in which a purine or a pyrimidine has been bound to a sugar via a β-N-glycosidic bond (ATP, GTP, CTP, UTP; or dATP, dGTP, dCTP or dTTP) have been bound to one another. Specific examples include a genomic DNA encoding a protein, an mRNA transcribed from the genomic DNA and a cDNA synthesized from the mRNA. It may be either a double strand or a single strand. Further, a sense strand and an antisense strand of these genomic DNA, mRNA and cDNA are included. In addition, 'protein' and 'peptide' mean a molecule composed of a plural number of amino acid residues bound one another via an amide bond (peptide bond).

The terms and concepts in this invention will be defined in detail in the description of the embodiments or Examples of the invention. In addition, various techniques used for implementing this invention can be easily and surely carried out by those skilled in the art based on known literatures or the like except for the techniques whose sources are particularly specified. For example, preparation of a drug is described in Remington's Pharmaceutical Sciences, 18th Edition, ed. A. Gennaro, Mack Publishing Co., Easton, PA, 1990, and techniques of genetic engineering and molecular biology are described in Sambrook, and Maniatis, in Molecular Cloning: A Laboratory Manual, Cold Spring Harbor Laboratory Press, New York, 1989; Ausubel, F. M. et al., Current Protocols in Molecular Biology, John Wiley & Sons, New York, N.Y, 1995 and the like.

### Brief Description of Drawings

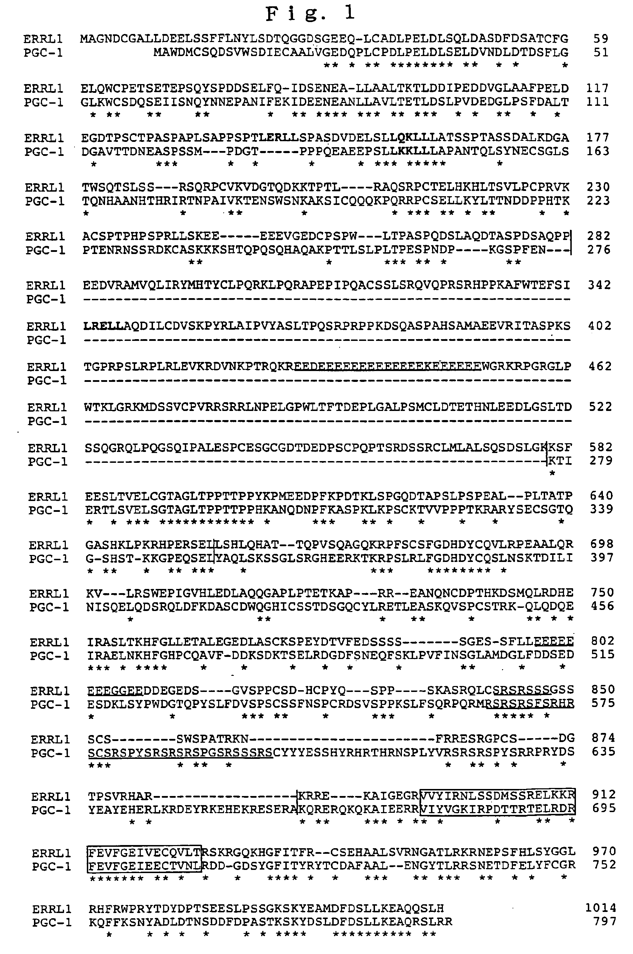

Fig. 1 shows the comparison of the primary amino acid sequences between ERRL1 and PGC-1. Asterisks indicate identical amino acids. The numbers represent the amino acid positions with the first methionine counted as one. LXXLL motifs are shown in bold types. Glutamic acid (E) repeats and serine-arginine (SR)-rich regions are underlined. A putative RNA-binding motif is boxed. Tentative domain borders are shown by vertical lines. A splicing variant of ERRL1, which was newly identified at this time, lacks 39 amino acids (from the 156th leucine to the 194th lysine).

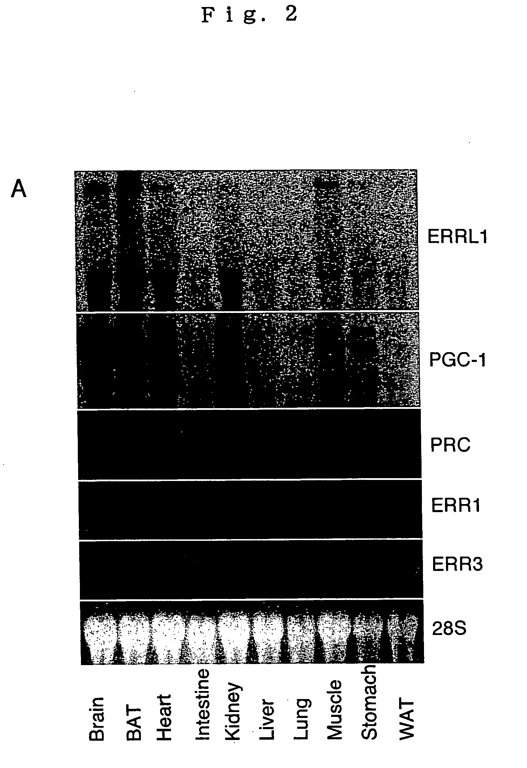

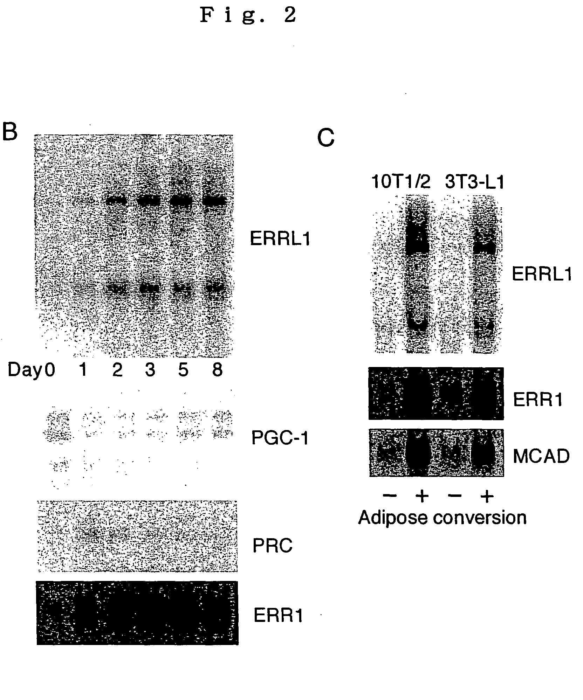

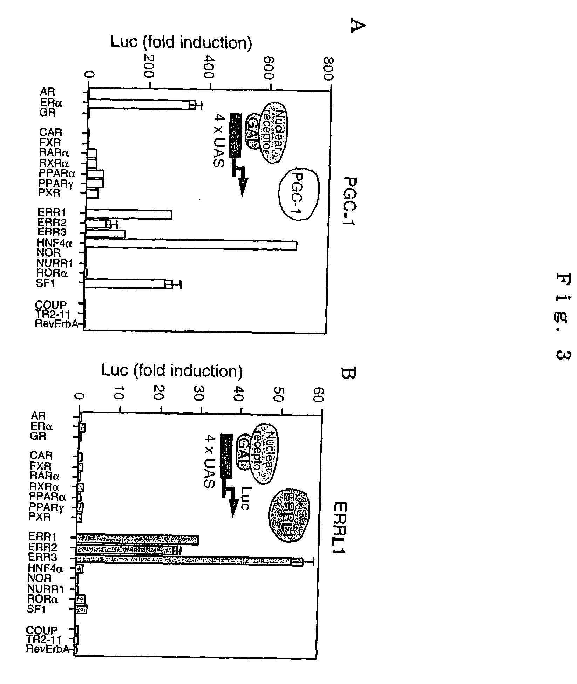

Fig. 2 shows the expression profiles of ERRL1 mRNA. Fig. 2A shows the results of analyzing 20 µg of total RNA from various adult mouse tissues using ERRL1, PGC-1, PRC, ERR1 and ERR3 cDNAs as probes. Ethidium bromide staining of 28S RNAs is also shown. Fig. 2B shows the results obtained by inducing 3T3-L1 cells to differentiate into adipocytes by the treatment with dexamethasone, 1-methyl-3-isobutylxanthin and insulin (added day 0), isolating RNAs and performing the Northern blot analysis. The blot was sequentially hybridized with the respective probes. Fig. 2C shows the results of observing the expression of ERRL1, ERR1 and MCAD mRNA in 3T3-L1 and 10T1/2 cells (-: preadipocytes, +: mature adipocytes).

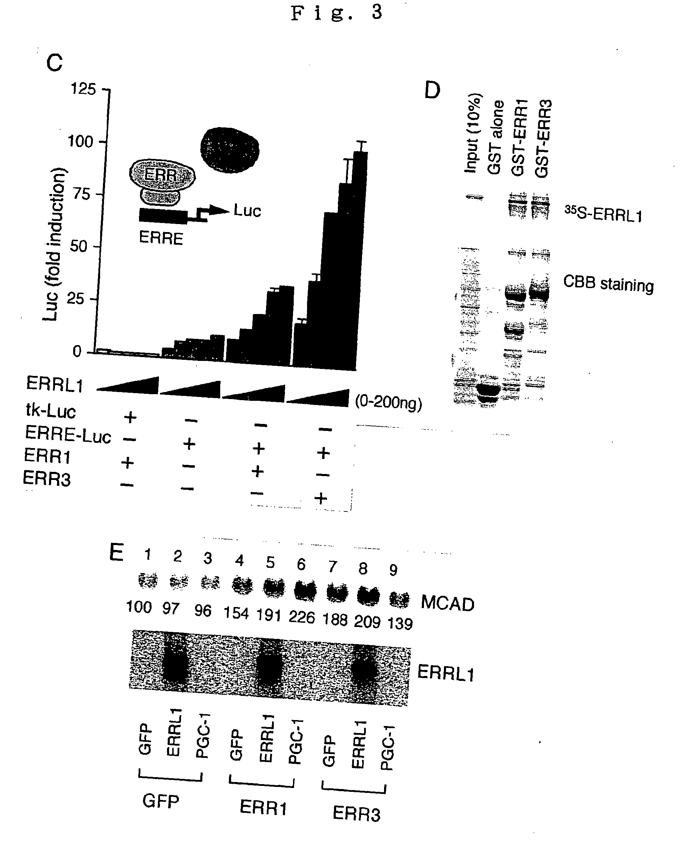

Fig. 3 shows the profiles of PGC-1 and ERRL1 as 'protein ligands' for various nuclear receptors. Transcriptional activation ability of PGC-1 (A) and ERRL1 (B) for various nuclear receptors was examined in a transfection assay. Mean values of triplicate experiments are shown as fold induction, where the Luc activity of each GAL4-fused nuclear receptor in the absence of PGC-1 or ERRL1 serves as the reference values. Error bars show standard deviation. Fig. 3C shows the dose-dependent activity of ERRL1 on the full-length ERR-mediated transcription via ERRE. pCMX-ERRL1 in different amounts (0 to 200 ng) was co-transfected with 30 ng of pCMX-ERR1 or pCMX-ERR3 in the presence of 250 ng of TK-Luc or ERRE-Luc in CV-1 cells. Mean values from triplicate experiments are shown as fold induction, where the Luc activity of TK-Luc in the absence of ERRL1 serves as the reference value. Error bars show standard deviation. Fig. 3D shows the results of confirming that ³⁵S-labeled ERRL1 shows strong interaction with GST-ERR1 and GST-ERR3 in vitro. Autoradiographs show the pulled down proteins (GST-ERR1 and GST-ERR3) and 10% of the total amount of ³⁵S-labeled ERRL1 used for the GST pull-down assay (Input). CBB staining of the SDS-PAGE gel containing GST-fused proteins used in this assay is also presented. Fig. 3E shows the results showing augmentation of MCAD mRNA expression by ectopic expression of ERRL1 or PGC-1 in combination with ERR1 or ERR3 in 10T1/2 cells. One day after confluence of the cells, total RNA was isolated, and analyzed by Northern blot (20 µg per lane) using MCAD and ERRL1 cDNAs as probes. The relative densitometric values of MCAD mRNAs are also included beneath each lane.

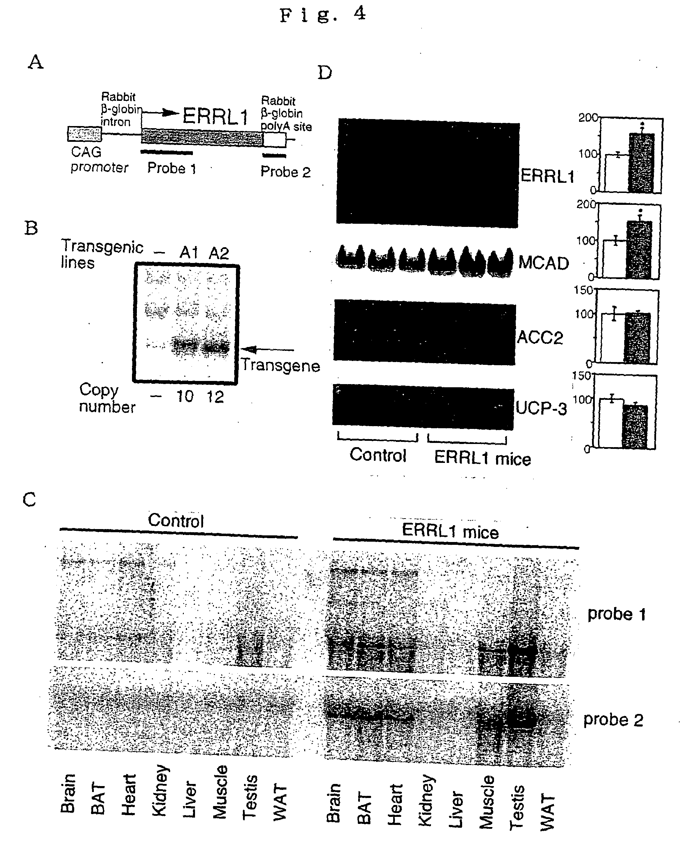

Fig. 4 relates to the creation of ERRL1 transgenic mouse. Fig. 4A shows the schematic drawing of the ERRL1 transgene and the positions of probes used for Southern (probe 1) and Northern blots (probe 1 and probe 2). Fig. 4B shows the results of showing that in the two transgenic lines (A1 and A2), 10 and 12 copies of each of the transgenes were contained, respectively. Fig. 4C shows the results of Northern blot analysis of ERRL1 mRNA expression in the respective tissues from ERRL1 mice (line A1) and littermate control mice. Each lane contained 20 µg of total RNA. Fig. 4D shows the results of measuring the expression of ERRL1, MCAD, ACC2 and UCP-3 in the skeletal muscle of ERRL1 transgenic mice and wild-type control mice. Three mice in each group were examined. Quantitative values of Northern signals (control as 100%) are shown on the right. Data are mean ± s.e.m. (Asterisk shows p < 0.05).

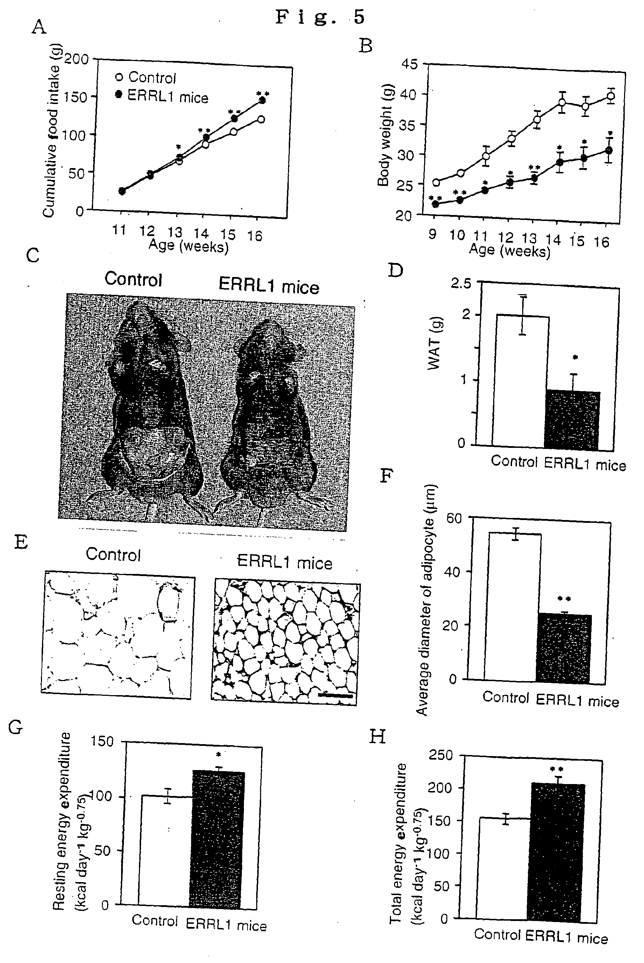

Fig. 5 relates to the phenotypes of ERRL1 transgenic mice. Fig. 5A shows the cumulative food intake per mouse during the indicated period by measuring food intake every week. Data are means ± s.e.m. (n = 6 per animal group, error bars are smaller than the symbols, asterisk shows P < 0.05, double asterisk shows P < 0.01). Fig. 5B shows the results of showing the changes in the body weight of ERRL1 transgenic mice (closed circle) and wild-type controls (open circle). Values represent mean body weight ± s.e.m. (n = 6 per animal group, asterisk shows P < 0.05, double asterisk shows P < 0.01). For some data points, the error bars are smaller than the symbols. Fig. 5C shows the results of reduction of abdominal fat in ERRL1 transgenic mice. Fig. 5D shows the comparison of epididymal WAT weight between wild-type control mice and ERRL1 transgenic mice. The columns represent mean values of WAT weight ± s.e.m. (n = 6 per animal group, asterisk shows P < 0.05). Fig. 5E shows the comparison of the morphology of white adipose tissue between ERRL1 transgenic mice and littermate wild-type control mice. The scale bar indicates 50 µm. Fig. 5F shows the average diameter of adipose cells. The diameters of the cells were measured from the sections shown in Fig. 5E (n = 20, double asterisk shows P < 0.01). Figs 5G and 5H show the results of showing energy expenditures at resting (G) and total (H) in 12-week-old control mice and ERRL1 transgenic mice. The columns represent mean values of energy expenditure ± s.e.m. (n = 6 per animal group, asterisk shows P < 0.05, double asterisk shows P < 0.01).

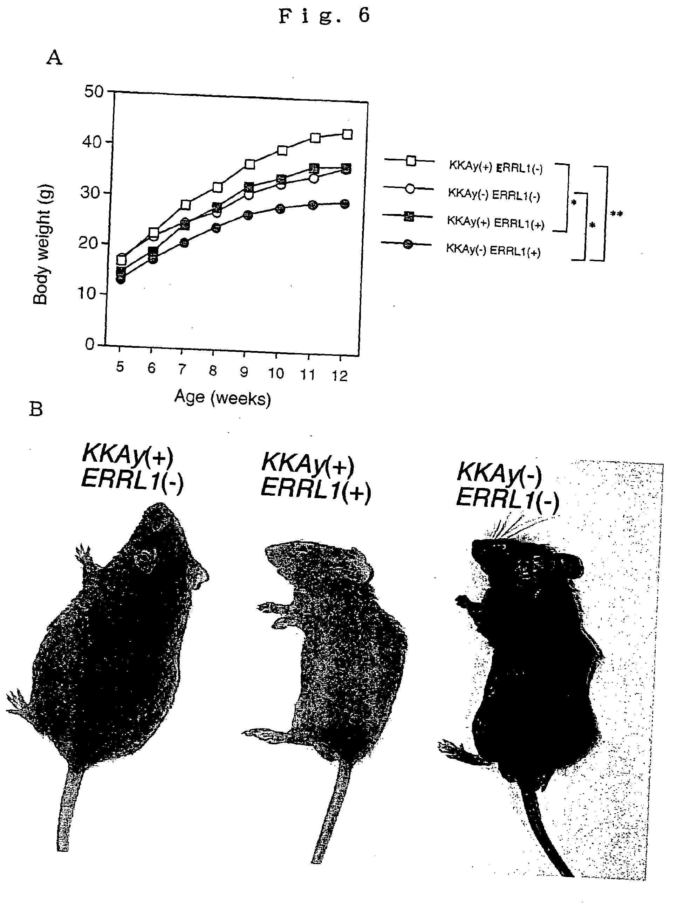

Fig. 6A shows the results of measuring changes in body weight of KKAy(+)ERRL1(-) (open square, n = 8), KKAy(-) ERRL1(-) (open circle, n = 5), KKAy(+)ERRL1(+) (closed square, n = 5), KKAy(-)ERRL1(+) (closed circle, n = 5) male mice. The body weight curve of each group was compared by repeated-measure analysis (Super ANOVA). Asterisk shows P < 0.05, double asterisk shows P < 0.01. Fig. 6B shows representative photographs of 12-week-old male KKAy(+)ERRL1(-) (left), KKAy(+)ERRL1(+) (center) and KKAy(-)ERRLl(-) (right) mice.

### Best Mode for Carrying Out the Invention

The ligand factor ERRL1 in the current invention is, for example, mouse ERRL1 (SEQ ID NO: 2) which has been disclosed as 'mouse PGC2' in SEQ ID NO: 1 in JP-A-2002-058489. Further, as for this ligand factor ERRL1, its splicing variant (the one lacking 39 amino acids from the 156th leucine to the 194th lysine of SEQ ID NO: 2) can be used as a target. In addition, known human PERC (PPARγ coactivator β1: Entrez protein database No. NP_573570), which is a human homologue to mouse PGC2, can be also used as a target of this invention for a human ERRL1. As the polynucleotide encoding mouse ERRL1, mouse PGC2 cDNA (ERRL1 cDNA: SEQ ID NO: 1) whose nucleotide sequence has been disclosed in SEQ ID NO: 2 in JP-A-2002-058489 can be used. Further, as the polynucleotide encoding human ERRL1, the foregoing human PERC cDNA (GenBank No. NM_133263) can be used. Still further, for example, human PRRC gene exists in chromosome 5q33.1 region, and its genomic sequence is publicly known (GenBank No. NT_029289). Therefore, this invention can be carried out by utilizing a promoter region existing in this genomic gene sequence.

With regard to the nuclear receptor ERR of this invention, any one or more selected from ERR1, ERR2 and ERR3 (Giguere, V. et al., Nature 331: 91-94, 1988; Shigeta, H. et al., J. Mol. Endocrinol. 19: 299-309, 1997; Eudy, J. D. et al., Genomics 50: 382-384, 1998; Hong, H. et al., J. Biol. Chem. 274: 22618-22626, 1999) can be used as a target. In addition, cDNAs encoding them, respectively, are also publicly known. For example, ERR1 cDNA is known on GenBank No. NM_004451, and the genomic gene (chromosome 11q13 region) sequence is known on GenBank No. NT_033903. Therefore, this invention can be carried out by using these polynucleotides (cDNA, genomic DNA, a DNA fragment of the gene promoter region and the like). Further, as for MCAD gene, its cDNA is also publicly known (GenBank No. AF251043).

The foregoing polynucleotides can be isolated by screening a genomic DNA library or a cDNA library using, as a probe, a DNA fragment consisting of a known nucleotide sequence or its partial sequence, respectively. The obtained polynucleotide can be amplified by a gene amplification method, which is usually performed, such as the PCR (polymerase chain reaction) method, NASBN (nucleic acid sequence based amplification) method, TMA (transcription- mediated amplification) method or SDA (strand displacement amplification) method. In addition, in the case of cDNA, by using a primer synthesized based on a known sequence, a target cDNA can be obtained by also the RT-PCR method in which mRNA isolated from the cell is used as a template.

The method of screening a substance which serves as the active ingredient in a drug for obesity and/or diabetes of this invention comprises treating cells or an animal with a candidate substance and specifying a candidate substance fulfilling one or more of the following requirements as the target substance by using the foregoing protein factors or polynucleotides encoding them:
(a) increasing the expression level of a ligand factor ERRL1;
(b) increasing the transcriptional activity of a nuclear receptor ERR;
(c) promoting the binding of ERRL1 to ERR; and
(d) increasing the expression level of an MCAD gene product.

In other word, as explained above, a nuclear receptor ERR serves as a regulator for MCAD gene expression, and energy balance in the body is controlled by regulating fatty acid β-oxidation by this MCAD gene expression, whereby the resistance to obesity or diabetes can be maintained. As shown in the Examples described later, ERRL1 is a ligand molecule for the receptor ERR which is the origin of a series of such mechanisms. Indeed, it was confirmed that augmentation of ERRL1 expression shows a significant effect on acquisition of the resistance to obesity or diabetes in mouse. In other words, a substance fulfilling the foregoing requirements (a) to (d) is a substance which acts on the process of anti-obesity and anti-diabetic mechanism.

In the methods of this invention, a 'candidate substance' includes an organic or inorganic substance, a protein, a peptide, a polynucleotide, an oligonucleotide and the like, which are either unknown or known. 'Cells' are ones isolated from an established human cell line or non-human animal cell line or from a tissue of a human or a non-human animal body and appropriately maintained in a culture condition. Examples of the established cell line include an adipocyte such as 3T3-L1 cell line. As described in Examples below, because the expression of ERRL1 protein shows a sensitive response during the differentiation of this adipocyte, those are preferred cells for the search for a target substance. Alternatively, the cells may be an established cell line or bacteria (E. coli or the like) transfected with ERRL1 cDNA and/or ERR cDNA. In addition, as the cells isolated from an animal body, those of isolated from BAT, heart, skeletal muscle, kidney or the like, in which ERRL1 protein has been highly expressed, can be exemplified. On the other hand, in the case of using a non-human animal (such as mouse) as a target, a candidate substance is administrated to this animal body (systemically or locally) or is taken as food or drink, then the foregoing requirements (a) to (d) are measured in a tissue or cells of the animal. At this time, a transgenic non-human animal provided by this invention may be used as a positive control. In other words, because this transgenic non-human animal overexpresses ERRL1 protein and, as a result, highly expresses receptor ERR and MCAD genes, therefore, by measuring whether or not a wild-type mouse administered with a candidate substance fulfills the foregoing requirements as much as this transgenic animal does, an effect of the candidate substance can be judged more precisely. In addition, in the case of using a transgenic non-human animal or a wild-type animal as a target, a treatment of feeding with a high-fat diet, loading with physical exercises or the like, or amount of food intake, body weight, energy expenditure or the like can be included in the judging requirements.

The judgment of the foregoing requirements (a) to (d) can be carried out by measuring the expression of the respective genes by measuring the level of their transcriptional products (mRNA or protein) using a known method. For example, the judgment of the foregoing requirements (a) to (d) can be carried out by a known method such as in situ hybridization, Northern blotting, dot blotting, RNase protection assay, RT-PCR, Real-time PCR (Journal of Molecular Endocrinology, 25, 169-193 (2000) and the literatures cited therein), and DNA array analysis method (edited by Mark Shena, "Microarray Biochip Technology", Eaton Publishing (March 2000)). A screening of an active ingredient substance in a drug for obesity and/or diabetes using such a technique and a reagent, method, process, analysis program and the like to be used therein are all included in this invention. Specific examples of the judgment include, for example, the following methods.

### (A) Screening of substance that increases expression level of ligand factor ERRL1

A reporter plasmid is constructed in which the promoter region of ERRL1 gene has been fused with cDNA of luciferase or β-galactosidase. After this reporter plasmid is transfected into cells, a candidate substance to be investigated is added to the cells and the activity of luciferase or β-galactosidase in the extracted solution of the cultured cells is measured after several days. If an increase of luciferase or β-galactosidase is observed by the addition of the candidate substance, the possibility is strongly suggested that this substance has an ability to activate the promoter of ERRL1 gene. Then, this candidate substance is added to cultured cells and an increase of mRNA expression level of ERRL1 gene is evaluated by the Northern blot method, quantitative RT-PCR method or the like. Similarly, using cultured cells, an increase of expression level of ERRL1 protein can be also evaluated by the Western blot method using an anti-ERRL1 antibody. Alternatively, a candidate substance is fed to a non-human animal and an increase of ERRL1 mRNA level or ERRL1 protein level in the cells isolated from the animal (e.g., BAT, heart, skeletal muscle, kidney or the like) may be evaluated in the same manner.

### (B) Screening of substance that increases the transcriptional activity of nuclear receptor ERR

After the reporter plasimd of luciferase or β-galactosidase having an ERR responsive sequence or gal4 responsive sequence in the promoter region is cotransfected into cultured cells with an expression vector of the full-length ERR protein or a fused protein of the ligand binding region of ERR with the DNA binding region of gal4, respectively, a candidate substance to be investigated is added to the cells and the activity of luciferase or β-galactosidase in the extracted solution of the cultured cells is measured after several days. If an increase of luciferase or β-galactosidase is observed depending on the existence of the responsive sequence by the addition of the candidate substance, the possibility is strongly suggested that this substance has an ability to activate the transcription of ERR gene. Then, this candidate substance is added to cultured cells and an increase of mRNA expression level of a target gene of ERR, for example MCAD gene, is evaluated by the Northern blot method, quantitative RT-PCR method or the like. Similarly, using cultured cells, an increase of expression level of the product of a target gene of ERR, for example MCAD protein, can be evaluated by the Western blot method using an anti-MCAD antibody. Alternatively, a candidate substance is fed to a non-human animal and an increase of MCAD mRNA level or MCAD protein level in the cells isolated from the animal (e.g., BAT, heart, skeletal muscle, kidney or the like) may be evaluated in the same manner.

### (C) Screening of substance that promotes binding of ERRL1 to ERR

In yeast or cultured animal cells, using the two hybrid method, the binding of ERRL1 to ERR is quantified as the activity of luciferase or β-galactosidase. When a candidate substance to be investigated is added to the system, if luciferase or β-galactosidase is increased compared to the case where the substance has not been added, the possibility is strongly suggested that this substance has an ability to activate the binding of ERRL1 to ERR. Then, when this drug substance is added in an immunoprecipitation method or a GST-pull down assay, the level of coprecipitated ERRL1 and ERR proteins is evaluated by the Western blot method. On the other hand, in a gel retardation assay using ERR protein and an oligo DNA containing the ERR responsive sequence, according to the index that is based on whether or not the level of signal that super shifts in the presence of ERRL1 is increased by the addition of this candidate substance, it can be confirmed whether or not this substance has an effect on promoting the binding of ERRL1 to ERR. Further, by adding this candidate substance to a detection assay system to detect interaction using an apparatus such as Biacore, it can be confirmed whether or not this substance has an effect on promoting the binding of ERRL1 to ERR.

### (D) Screening of substance that increases expression level of MCAD gene product

A reporter plasmid is constructed in which the promoter region of MCAD gene has been fused with cDNA of luciferase or β-galactosidase. After this reporter plasmid is transfected into cells, a candidate substance to be investigated is added to the cells and the activity of luciferase or β-galactosidase in the extracted solution of the cultured cells is measured after several days. If an increase of luciferase or β-galactosidase is observed by the addition of the candidate substance, the possibility is strongly suggested that this substance has an ability to activate the promoter of MCAD gene. Then, this candidate substance is added to cultured cells and an increase of mRNA expression level of MCAD gene is evaluated by the Northern blot method, quantitative RT-PCR method or the like. Similarly, using cultured cells, an increase of expression level of MCAD protein can be evaluated by the Western blot method using an anti-MCAD antibody. Alternatively, by the same method as in the foregoing (B), an increase of MCAD mRNA level or MCAD protein level may be evaluated in a non-human animal.

The genetic engineering techniques (recombinant DNA techniques) used in each of the foregoing methods can be carried out in accordance with the methods described in J. Sambrook, E. F. Fritsch & T. Maniatis, "Molecular Cloning: A Laboratory Manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); Japanese Biochemical Society ed., "Zoku Seikagaku Jikken Kouza 1, Idenshi Kenkyuho II" Tokyo Kagaku Dozin (1986); Japanese Biochemical Society ed., "Shin Seikagaku Jikken Kouza 2, Kakusan III (Kumikae DNA Gijutsu)" Tokyo Kagaku Dozin (1992); R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987), J. H. Miller ed., "Methods in Enzymology", Vol. 204, Academic Press, New York (1991); R. Wu et al., ed., "Methods in Enzymology", Vol. 218, Academic Press, New York (1993); S. Weissman ed., "Methods in Enzymology", Vol. 303, Academic Press, New York (1999); J. C. Glorioso et al., ed., "Methods in Enzymology", Vol. 306, Academic Press, New York (1999), etc. or the methods described in the references cited therein or substantially the same methods or the modifications thereof.

The anti-ERRL1 antibody to be used in the foregoing method (A) and the anti-MCAD antibody to be used in the foregoing methods (B) and (D) are a polyclonal antibody or a monoclonal antibody that recognizes either ERRL1 protein or MCAD protein, and include a whole molecule that can bind to the epitope of the respective proteins, and all the fragments of Fab, F(ab')₂, Fv and the like. Such an antibody can be obtained from serum after immunizing an animal by using a purified ERRL1 protein or MCAD protein, or a partial peptide thereof as an antigen. Alternatively, it can be prepared by introducing an expression vector for a eukaryotic cell into the muscle or the skin of an animal with an injector or a gene gun, and collecting the serum. As the animal, mouse, rat, rabbit, goat, chicken or the like is used. If hybridoma is produced by fusing B cells collected from the spleen of an immunized animal with myeloma, a monoclonal antibody can be produced. Preparation of such an antibody can be carried out in accordance with a known method described in the literatures (Shin Seikagaku Jikken Kouza 12, Molecular Immunology III, Antigens, Antibodies, and Complements, Nancodo, 1992; "Monoclonal Antibody", co-authored by Komei Nagamune and Hiroshi Terada, Hirokawa Shoten, 1990; "Monoclonal Antibody", James W. Goding, third edition, Academic Press, 1996 and the like). Further, the measurement methods using such an antibody can be carried out by referring to the literatures, for example, Hiroshi Irie ed., "Radioimmunoassay" (published by Kodansha, 1974); Hiroshi Irie ed., "Radioimmunoassay; Second Series" (published by Kodansha, 1979); Eiji Ishikawa, et al. ed., "Enzyme Immunoassay" (published by Igaku Shoin, 1978); Eiji Ishikawa, et al. ed., "Enzyme Immunoassay" (Second Edition) (published by Igaku Shoin, 1982); Eiji Ishikawa, et al. ed., "Enzyme Immunoassay" (Third Edition) (published by Igaku Shoin, 1987); H. V. Vunakis et al. ed., "Methods in Enzymology", Vol. 70 (Immunochemical Techniques, Part A), Academic Press, New York (1980); J. J. Langone et al. ed., "Methods in Enzymology", Vol. 73 (Immunochemical Techniques, Part B), Academic Press, New York (1981); J. J. Langone et al. ed., "Methods in Enzymology", Vol. 74 (Immunochemical Techniques, Part C), Academic Press, New York (1981); J. J. Langone et al. ed., "Methods in Enzymology", Vol. 84 (Immunochemical Techniques, Part D: Selected Immunoassays), Academic Press, New York (1982); J. J. Langone et al. ed., "Methods in Enzymology", Vol. 92 (Immunochemical Techniques, Part E: Monoclonal Antibodies and General Immunoassay Methods), Academic Press, New York (1983); J. J. Langone et al. ed., "Methods in Enzymology", Vol. 121 (Immunochemical Techniques, Part I: Hybridoma Technology and Monoclonal Antibodies), Academic Press, New York (1986); J. J. Langone et al. ed., "Methods in Enzymology", Vol. 178 (Antibodies, Antigens, and Molecular Mimicry), Academic Press, New York (1989); M. Wilchek et al. ed., "Methods in Enzymology", Vol. 184 (Avidin-Biotin Technology), Academic Press, New York (1990); J. J. Langone et al. ed., "Methods in Enzymology", Vol. 203 (Molecular Design and Modeling: Concepts and Applications, Part B: Anibodies and Antigens, Nucleic Acids, Polysaccharides, and Drugs), Academic Press, New York (1991) and the like, or the references cited therein.

In the case where screening is carried out by using a non-human animal as a target in the foregoing methods (A), (B) and (D), a transgenic non-human animal provided by this invention can be used as a positive control. In other words, this transgenic non-human animal of this invention overexpresses ERRL1 protein and, as a result, it highly expresses receptor ERR and MCAD genes, thereby showing an anti-obesity and anti-diabetic property. Accordingly, the action of the candidate substance in the individual can be determined by using the gene expression in the cells of this transgenic animal as a control.

The transgenic non-human animal in this invention can be produced in accordance with a known transgenic animal production method (e.g., Proc. Natl. Acad. Sci. USA 77; 7380-7384, 1980). In other words, the foregoing mouse ERRL1 polynucleotide or human ERRL1 (human PERC) polynucleotide (hereinafter, referred to as 'introduced gene' in some cases) is introduced into a totipotent cell of the non-human animal and this cell is developed until it becomes an individual. Then, by selecting an individual in which the introduced gene has been integrated into the genome of the somatic cell, the target transgenic non-human animal can be produced. As the non-human animal, animals used as a laboratory animal such as mouse, rat, rabbit, dog, cat and the like can be used as a target, however use of mouse is preferred because the inbred line has been established. In addition, a promoter sequence or an enhancer sequence is connected to the introduced gene to control its expression. Depending on the selection of this promoter/enhancer sequence, ERRL1 protein can be expressed systemically or it can be selectively expressed in a specific tissue. Such an introduced gene can be constructed by introducing and ligating the foregoing polynucleotide or promoter/ enhancer sequence to a circular DNA vector so as to be arranged in an effective position in the expression regulation of the introduced gene. Then, after this vector DNA is digested with a restriction enzyme, the one, from which the vector region has been removed, is introduced into a totipotent cell. As the totipotent cell to which a gene is introduced, a fertilized egg, an early embryo or an embryonic stem cell (ES cell) can be used. In addition, as the method of introducing a gene into a totipotent cell, when a ratio of generating transgenic animal individuals or a ratio of inheriting an introduced gene to the next generations are taken into account, a physical injection (microinjection) method of DNA is the most preferred. A fertilized egg, to which a gene is injected, is then transferred to the oviduct of a surrogate mother and after it is generated to an individual and born, the animal is grown with a foster parent and DNA is extracted from part of the body (e.g., tail tip), and the existence of the introduced gene is confirmed by the Southern analysis or the PCR method. Assuming that the individual in which the existence of the introduced gene has been confirmed is the first generation (founder), the introduced gene is inherited to 50% of its offspring (F1). Further, by crossing this F1 individual with a wild-type animal or other F1 animal, an individual (F2) having the introduced gene in one (heterozygote) or both (homozygote) of diploid chromosomes can be produced. The transgenic animal produced as above overexpresses ERRL1 protein in all somatic cells or a specific tissue and, as described in the after-mentioned Examples, it has a unique characteristic of being lean although it is hyperphagic, and showing significantly high energy expenditure. Such a transgenic animal is useful not only as a control in the foregoing screening method but also as a model animal in the elucidation of an anti-obesity or anti-diabetic mechanism at the whole body level.

The invention of this application further provides a novel drug for obesity and/or diabetes comprising as the active ingredient at least one substance specified by the foregoing screening method. In other words, this drug has at least one of the actions selected from, firstly, the action of increasing the expression level of ERRL1, secondary, the action of increasing the transcriptional activity of ERR, thirdly, the action of increasing the interaction between ERRL1 and ERR, and fourthly, the action of increasing the expression level of an MCAD gene product.

The drug of this invention can be formulated by homogenously mixing the foregoing active ingredient and a pharmaceutically acceptable carrier. The carrier can be appropriately selected from a wide range according to the administration form of the drug. It is desired that the drug of this invention be in a unit dosage form that can be administered orally or by injection. The oral liquid preparation such as a suspension or syrup can be produced by using water, a sugar such as sucrose, sorbitol or fructose, a glycol such as polyethylene glycol, an oil such as sesame oil or soybean oil, an antiseptic such as alkyl parahydroxy benzoate, a flavor such as strawberry flavor or peppermint, or the like. A powder, pill, capsule and tablet can be formulated by using a diluent such as lactose, glucose, sucrose or mannitol, a disintegrating agent such as starch or sodium alginate, a lubricant such as magnesium stearate or talc, a binder such as polyvinyl alcohol, hydroxypropyl cellulose and gelatin, a surface active agent such as a fatty acid ester, an elasticizer such as glycerin or the like. The tablet and capsule are a preferred unit dosage form in the formulation of this invention in that it is easy to administer. When the tablet or capsule is produced, a solid pharmaceutical carrier is used. In addition, the solution for injection can be formulated by using a carrier composed of a salt solution, glucose solution, or a mixture of a salt solution and glucose solution, a variety of buffer solutions or the like. Further, an injectable solution may be also prepared by formulating it in a powder state and then mixing it with the foregoing liquid carrier prior to its use.

Hereunder, the invention of this application will be explained in more detail and specifically by showing Examples, however, the invention of this application is not intended to be limited to the following examples.

### Examples

### 1. Methods

### 1.1. Database search

An EST homology search was performed using the BLAST program (Altshul, S. F. et al., Nucleic Acids Res. 25: 3389-3402, 1997).

### 1.2. RNA analysis

Northern blot analyses were performed in accordance with the description in the literature (Sambrook and Maniatis, in Molecular Cloning-A Laboratory Manual, 7.2-7.87, Cold Spring Harbor Laboratory Press, New York, 1989). The cDNA probes for MCAD (Genbank accession No. U07159), PRC (BC013720), ERR1 (U85259), ERR2 (S82458), ERR3 (AF117254), ACC2 (AF290178), and UCP-3 (AF032902) were obtained by RT-PCR.

### 1.3. Assays for transcriptional activity

Transfections and reporter assays were performed in accordance with the description in the literature (Takada, I. et al., Mol. Endocrinol, 14: 733-740, 2000). More specifically, a reporter gene containing four copies of a GAL4 binding sequence ((UAS)₄-Luc) was transfected into CV-1 cells in the presence or absence of a chimeric receptor expression vector in which the DNA binding region of GAL4 had been fused with the ligand binding region for the nuclear receptor (pCMX-GAL4-nuclear receptor), and the transcriptional activity of GAL4-nuclear receptor-fused protein was measured (Takada, I. et al., Mol. Endocrinol, 14: 733-740, 2000). All luciferase activities were normalized by the co-transfected β-galactosidase activities. Amino acids 1-147 of GAL4 fused to the ligand binding domain for the following nuclear receptors were used: mouse AR (amino acids, 607-899, Genbank accession number, X59592), human ERα (a.a. 251-595, X03635), human GR (a.a. 489-777, M10901), rat FXR (a.a. 190-469, U18374), human RARα (a.a. 126-432, X06538), human RXRα (a.a. 222-462, X52773), mouse PPARα (a.a. 156-468, X57638), human PPARγ1 (a.a. 176-478, L40904), human PXR (a.a. 110-434, AF084645), human ERR1 (a.a. 147-422, L38487), human ERR2 (a.a. 171-433, X51417), human ERR3 (a.a. 173-436, AF058291), human HNF4α (a.a. 125-465, X76930), human NOR (a.a. 361-626, D78579), human NURR1 (a.a. 264-535, S77154), human RORα1 (a.a. 140-523, U04897), human SF1 (a.a. 64-461, U76388), human COUP (a.a. 156-423, X12795), human TR2-11 (a.a. 149-603, M29960), human RevErbA (a.a. 199-614, M24898). The abbreviations of nuclear receptors are described in the references of respective Genbank files (Giguere, V., Endocr. Rev. 20: 689-725, 1999). GAL4-nuclear receptor expression plasmids were provided from Dr. K. Umesono. Mouse PGC-1 cDNA was obtained by screening a mouse embryo cDNA library. The full length cDNA of human ERR1, 2 and 3 were obtained by RT/PCR. The amplified products were subcloned into pCAGGS and pCMX expression vectors, and confirmed by sequencing.

### 1.4. Protein interaction assays

In accordance with the description in the literature (Kamei, Y. et al., Cell 85: 403-414, 1996), the constructs (expression vectors) of GST fused with ERR1 and ERR3 were incubated with ³⁵S-ERRL1 (TNT, Promega) and washed. Then, the bound ³⁵S-ERRL1 was separated by SDS-PAGE, and quantified by autoradiography.

### 1.5. Stable cell lines

Phoenix 293 cells (gifted from Dr. G. P. Nolan, University of Stanford) were used for the retrovirus packaging (Grignani, F. et al., Cancer Res. 58: 14-19, 1998). pLNCX-derived expression plasmid (Clontech) containing cDNA of ERR1, ERR3 or GFP (control) and pMX-derived expression plasmid (Misawa, K. et al., Proc. Natl. Acad. Sci. USA 97: 3062-3066, 2000) containing cDNA of ERRL1, PGC-1 or GFP were used in accordance with the manufacturer's instruction.

### 1.6. Generation of transgenic mice

ERRL1 cDNA was cloned into pCAGGS (Niwa, H., Gene 108: 193-200, 1991), the transgene (Fig. 4A) was excised and purified (2 ng µl⁻¹). Fertilized eggs were recovered from BDF1 females (C57BL/6xDBA/2) crossed with BDF1 males and microinjected with the transgene by the standard method (Gordon, J., in Guide to Techniques in Mouse Development, (eds Wassarman, P. M. & DePamplilis, M. L., 747-771, Academic press, San Diego, 1993). Care of the mice was performed in accordance with the guideline of the institution to which the inventors belong.

### 1.7. High-fat diet

Mice were fed with a regular chow diet (Oriental Yeast Inc., Tokyo, Japan) or a high-fat diet (Aoki, N. et al., Obesity Res. 1: 126-131, 1993) which contained casein (20% wt/wt), α-cornstarch (30.2%), sucrose (10%), lard (25%), corn oil (5%), minerals (3.5%), vitamins (1%), cellulose powder (5%), and D,L-methionine (0.3%).

### 1.8. Determination of energy expenditure

Oxygen consumption and carbon dioxide production were determined by using an indirect calorimeter system composed of a mass analyzer and a computer (Komenani, N. et al., J. Nutr. Sci. Vitaminol. (Tokyo) 41: 395-407, 1995). Mice were individually placed in an open-circuit plastic respiration chamber (24 x 46 x 18 cm) connected to a gas mass spectrometer (WSMR-1400, Westron, Chiba, Japan). The airflow was controlled at 2 1/min. Gas analysis was carried out from 10:00 to 9:00 on the following day. Samples were monitored continuously in 2 minute blocks in room air as a reference. Locomotor activity was recorded automatically every 10 minutes by an Animex-III (Shimadzu, Kyoto, Japan) laid under each respiration chamber. Energy expenditure was calculated in accordance with the description in the literature (Komenani, N. et al., J. Nutr. Sci. Vitaminol. (Tokyo) 41: 395-407, 1995).

### 1.9. KKAy mice

KKAy mice were purchased from Clea Japan Inc. (Tokyo).

### 1.10. Statistical analyses

Statistical comparisons of data from two experimental groups were made by using Student's t-test. Comparison of data from multiple groups was made by one-way analysis of variance (ANOVA), and each group was compared with the other groups by the Fisher's protected least significant difference (PLSD) test (Statview 4.0, Abacus Concepts). Statistical significance was defined as P < 0.05 or P < 0.01.

### 2. Results

### 2.1. Cloning of ERRL1 cDNA and its features

As a result of searching EST (expression sequence tags) for a PGC-1-related molecule, EST having an extremely high homology to PGC-1 was found, and the full length cDNA containing this EST was isolated. This cDNA consists of about 3.4 kb (SEQ ID NO: 1), and encodes a protein consisting of 1,014 amino acid sequences (sequence number 2). This protein was named ERRL1 (abbreviation of ERR ligand 1) based on its properties as a 'protein ligand' for ERRs (as shown later in detail). Incidentally, Lin et al. reported the cloning of a PGC-1 homologue named PGC-1β (Lin, J. et al., J. Biol. Chem. 277: 1645-1648, 2002), however, PGC-1β only has one amino acid difference from ERRL1 (The 260th leucine in the sequence number 2 is a proline in the PGC-1β). ERRL1 and PGC-1 show a high degree of amino acid identity except for the central unique region in ERRL1. The homologous region can be divided into five domains based on sequence identity and predicted functional properties (Fig. 1). The N-terminal region of ERRL1 (amino acids 1-282) contains two LXXLL motifs, which are a proposed binding motif to nuclear receptors (Torchia, J. et al., Nature, 387: 677-684, 1997; Heery, D. M., et al., Nature, 387: 733-736, 1997), and has 41% identity with PGC-1. The second region (amino acids 283-579), which is unique to ERRL1, contains E (glutamic acid) repeats and one LXXLL motif. The third region (amino acids 580-656) is highly conserved (47% amino acid identity). The fourth region (amino acids 657-882) is less conserved (22% amino acid identity) and contains a very short serine/arginine (SR)-rich domain (Tacke, R. & Manley, J. L. Curr. Opin. Cell Biol. 11: 358-362, 1999) in ERRL1 compared with the long SR domain in PGC-1. The C-terminal domain (amino acids 883-1014) has a putative RNA-binding domain (Krecic, A. M. & Swanson, M. S., Curr. Opin. Cell Biol. 11: 363-371, 1999) and is highly conserved (52% amino acid identity).

### 2.2. Similar expression patterns between ERRL1 and ERR

The expression patterns of ERRL1 in different tissues from adult mice were examined. Two ERRL1 mRNAs of about 10 kb and 4 kb in length were observed. ERRL1 mRNAs were abundant in brain, BAT, heart and skeletal muscle, and were also detected in kidney, stomach and white adipose tissue (WAT). Consistent with a previous report (Puigserver, P. et al., Cell 92: 829-839, 1998), PGC-1 expression was increased in mouse BAT after exposure to cold stress, however, the expression of ERRL1 mRNAs was only marginally up-regulated by this stress.

Further, the expression pattern of ERRL1 closely resembles that of ERR1 (Sladek, R. et al., Mol. Cell. Biol. 17: 5400-5409, 1997 and Fig. 2), with both mRNAs being highly expressed in tissues that can use lipids as a source of cellular energy, e.g., BAT, heart, skeletal muscle, and kidney (Fig. 2A). ERR2 mRNA expression was not be detected, which is consistent with a previous study report (Giguere, V. et al., Nature 331: 91-94, 1988).

Then, it was examined whether ERRL1 expression is augmented during adipocyte differentiation of 3T3-L1 cells. It is certain that ERRL1 mRNAs were present at very low levels in 3T3-L1 preadipocytes and were markedly induced during adipocyte differentiation. In contrast, mRNAs of PGC-1 and PRC, another PGC-1-related molecule (Andersson, U. & Scarpulla, R. C. Mol. Cell. Biol. 21: 3738-3749, 2001) remained at low levels during this adipocyte differentiation (Fig. 2B). Similar augmentation of ERRL1 mRNAs was observed in mature adipocytes (Smas, C. M. & Sul, H. S. Biochem. J. 309: 697-710, 1995) differentiated from another preadipocytes, 10T1/2 cells (Fig. 2C). Further, expression profiles of ERR1 and its target MCAD were quite similar to that of ERRL1 (Figs. 2B and 2C), however, ERR2 mRNA and ERR3 mRNA expressions were not detected in these cells. These results suggest that ERRL1 is involved in the gene regulations in mature adipocytes-related functions such as the lipid metabolism.

### 2.3. PGC-1 as a general nuclear receptor agonist.

It was examined that whether ERRL1 can function as a coactivator of PPARγ, using PGC-1 as a control coactivator. Contrary to our expectations, any evidence indicating that ERRL1 could activate PPARγ-mediated transcription could not be found out. In the control experiments, it was confirmed that the expression of PGC-1 on its own was able to drastically activate PPARγ-mediated transcription without the addition of any exogenous lipophilic ligands. PGC-1 is known to physically interact not only with PPARγ but also with several other nuclear receptors (Puigserver, P. et al., Cell 92: 829-839, 1998; Yoon, J. C. et al., Nature 413: 131-138, 2001; Tcherepanova, I. et al., J. Biol. Chem. 275: 16302-16308, 2000; Vega, R. B. et al., Mol. Cell. Biol. 20: 1868-1876, 2000; Delerive, P. et al., J. Biol. Chem. 277: 3913-3917, 2002). Furthermore, PGC-1 has been shown to be induced by certain environmental changes such as cold exposure (Puigserver, P. et al., Cell 92: 829-839, 1998) and fasting (Yoon, J. C. et al., Nature 413: 131-138, 2001; Lehman, J. J. et al., J. Clin. Invest. 106: 847-856, 2000). These observations raise the possibility that the expression of PGC-1 alone can activate multiple nuclear receptor pathways. This putative character is analogous to classical lipophilic ligands for the nuclear receptors. To address this possibility, nuclear receptors whose DNA-binding domains were replaced by the GAL4 DNA-binding domain were used. By this replacement, the comparison of transcriptional activity profiles of multiple nuclear receptors with the same reporter (gene) such as (UAS)₄-Luc can be easily performed. Indeed, PGC-1 was able to activate transcription by means of multiple nuclear receptors including orphan HNF4α, SF1, and ERRs. Among these, PGC-1 most strongly activated HNF4α, which is consistent with a recent report (Yoon, J. C. et al., Nature 413: 131-138, 2001). Subsequently, PGC-1 activated ERα, SF1, ERRs, PPARs, PXR, RARα, and RXRα (Fig. 3A). From these observations, it was confirmed that PGC-1 can function as a general and broad nuclear receptor agonist.

### 2.4. ERRL1 as an ERR protein ligand

Using the same GAL4-fused nuclear receptor set as above, a potential partner of ERRL1 was searched, and it was found out that ERRL1 specifically activated ERR-mediated transcription. ERR3 was most strongly activated by ERRL1, followed by ERR1 and ERR2 (Fig. 3B). Then, the transcriptional activation properties of ERRL1 on full-length ERRs were tested. Consistent with the experiments above, ERRL1 dose-dependently activated the full-length ERR3- and the full-length ERR1-mediated transcription by means of the ERR responsive sequence (ERRE) of the MCAD gene promoter (Sladeck, R. et al., Mol. Cell. Biol. 17: 5400-5409, 1997; Vega, R. B. & Kelly, D. P. J. Biol. Chem. 272: 31693-31699, 1997) (Fig. 3C). The former was activated much more strongly than the latter.

Next, it was tested whether ERRL1 physically interacts with ERRs by in vitro binding analysis with glutathione S-transferase-fused ERR (GST-ERR) produced by using bacteria and ERRL1 translated in vitro. Matrix-bound GST-ERR1 and GST-ERR3, but not GST alone, retained radiolabeled ERRL1 efficiently (Fig. 3D). As reported previously (Sladek, R. et al., Mol. Cell. Biol. 17: 5400-5409, 1997; Vega, R. B. & Kelly, D. P. J. Biol. Chem. 272: 31693-31699, 1997), it was confirmed that ERRs had been bound to radiolabeled MCAD ERRE oligonucleotides in a gel mobility shift assay, and that the ERR-DNA complexes were supershifted by the addition of the ERRL1 protein. Given that lipophilic hormones were not added either in the synthesis of the proteins or in the binding reactions, these results indicate that ERRL1 and ERRs can directly interact with the ERR target promoter DNAs.

Next, ERRL1 and ERR were expressed in 10T1/2 cell line, and it was examined whether ERRL1 can activate MCAD gene expression via the ERR. 10T1/2 cells were infected with several combinations of recombinant retroviruses, each containing a DNA encoding GFP (control), ERR1, ERR3, ERRL1 or PGC-1. Northern blot analysis was performed to confirm the expressions of the transduced genes in each cell line (Fig. 3E). The sole expression of either ERR1 or ERR3 led to a modest increase of MCAD mRNA level compared with that in cells infected with GFP alone. Coexpression of both ERR1 and ERRL1 or ERR3 and ERRL1 led to a further significant increase of the MCAD mRNA level (Fig. 3E). These results collectively demonstrate that ERRL1 can function as a 'protein ligand' for ERRs and activate ERR-mediated transcription at least in cultured cells.

### 2.5. Creation of ERRL1 mice

In order to examine the effects of increased ERRL1 expression in vivo, ERRL1 transgenic mice were created. It was expected that these mice would exhibit phenotypes that mimic those induced by activated ERR-mediated transcription. The chicken β-actin promoter with the cytomegalovirus immediate early enhancer (CAG promoter) was used to promote the expression of the mouse ERRL1 transgene in mice (Fig. 4A). Southern blot analysis of tail DNAs of the mice was performed to determine the transgene copy numbers (Fig. 4B).

Expression of the ERRL1 transgene was evaluated by Northern blot analysis on RNAs isolated from tissues of ERRL1 mice and control littermate mouse at 8 weeks of age (Fig. 4C). When a transgene-specific probe (Probe 2, Fig. 4A) was used, only a single 4-kb band was detected. The CAG promoter has been reported to show a strong activity in any tissues (Niwa, H. et al., Gene 108: 193-200, 1991). Unexpectedly, however, the use of this promoter resulted in high expression levels of the ERRL1 transgene in several restricted tissues such as brain, BAT, heart, skeletal muscle, and testis, but a very low-level of expression in liver (Fig. 4C). These expression profiles corresponded relatively well to the endogenous ERRL1 expression patterns (Fig. 4C). These observations suggest that tissue-specific regulatory cis-sequences crucial for ERRL1 expression may reside in the ERRL1 cDNA used in this construct.

As predicted from the in vitro data, the increased expression of ERRL1 in the skeletal muscle of ERRL1 mice (Fig. 4D) enhanced MCAD mRNA expression in vivo. In contrast, the increased expression of ERRL1 did not affect the gene expression of acetyl-CoA carboxylase 2 (ACC2) (Abu-Elheiga, L. et al., Science 291: 2613-2616, 2001) or uncoupling protein-3 (UCP3) (Clapham, J. C. et al., Nature 406: 415-418, 2000).

### 2.6. ERRL1 mice are hyperphagic but lean

The apparent phenotype observed in ERRL1 mice was lean, which agreed well with those predicted from increased β-oxidation of fatty acids. This phenotype was more prominent when mice were fed with a high-fat diet (Aoki, N. et al., Obesity Res. 1: 126-131, 1993). Groups of six male wild-type control mice and six male ERRL1 mice, at 9 weeks of age, were freely fed with a high-fat diet, and the food consumption and body weight of each mouse were measured every week. ERRL1 mice consumed significantly more food than the control mice (Fig. 5A). However, the transgenic mice weighed 15 to 25% less than the control mice before and throughout the feeding periods with a high-fat diet (Fig. 5B), and they accumulated significantly less fat in their adipose tissues (Fig. 5C). The epididymal WAT in the ERRL1 mice only weighed 0.92 ± 0.28 g, compared with 2.0 ± 0.30 g in the control mice (Fig.). In contrast, liver weights were not significantly different (ERRL1 mice: 1.23 ± 0.06 g; control mice: 1.4 ± 0.08 g). The adipocytes of the transgenic mice were smaller than those of the control mice (average diameter of adipocytes in ERRL1 mice: 25.1 ± 1.1 µm; control mice: 54.5 ± 2.2 µm; Figs. 5E and 5F).

The blood was collected from these mice, and serum components were biochemically analyzed. The decrease in adipose mass resulted in a decrease in the amount of leptin in the serum (ERRL1 mice: 5.9 ± 3.5 ng ml⁻¹, control mice: 25.0 ± 6.6 ng ml⁻¹). As leptin is an anti-appetite hormone secreted from WAT (Friedman, J. M. Nature 404: 632-634, 2000), the decreased level of leptin observed in ERRL1 mice is consistent with the observation that they are hyperphagic. Furthermore, decreased level of insulin were observed in the transgenic mice compared with the control mice fed with a high-fat diet. Mice, when fed with a high-fat diet, generally develop resistance to glucose uptake, even with a high plasma insulin concentration (Li, B. et al., Nat. Med. 6: 1115-1120, 2000). A similar state, which is called insulin resistance, is well observed in humans with obesity or who are suffering from type 2 diabetes (Lovejoy, J. C. Curr. Atheroscler. Rep. 1: 215-220, 1999). The decreased body weight and decreased insulin level observed in ERRL1 mice indicate that the increased expression of ERRL1 can antagonize obesity and contribute to an improvement of diabetic state, even taking a high-calorie diet through overcoming insulin resistance.

### 2.7. Increased energy expenditure of ERRL1 mice

The energy expenditure of ERRL1 mice was examined. Gas analyses were carried out by using test chambers for respiration analysis, each containing a single mouse, and energy expenditure was calculated. As a result, the energy expenditures of ERRL1 mice were significantly higher than those of control mice at 12 weeks of age (resting energy expenditure: ERRL1 mice, 126.3 ± 3.8 kcal day⁻¹ kg^{-0.75}; control mice, 101.5 ± 6.7 kcal day⁻¹ kg^{-0.75}; P < 0.05, n = 6, per group; and total energy expenditure: ERRL1 mice, 211.2 ± 10.7 kcal day⁻¹ kg^{-0.75}; control mice, 155.9 ± 7.8 kcal day⁻¹ kg^{-0.75}; P < 0.01, n = 6, per group) (Figs. 5G and 5H). However, locomotor activity was not significantly different (ERRL1 mice, 7,338 ± 700 counts; control mice, 5,338 ± 390 counts, the data represent the sum of all transits over a 24 h period). These results indicate that the differences in body weights between the control mice and the ERRL1 mice are most likely to be due to a difference in their energy expenditure.

### 2.8. ERRL1 expression can antagonize a genetically programmed obesity

ERRL1 mice and KKAy mice were bred, and it was examined whether the obese phenotype of KKAy mice was counteracted by the increased expression of ERRL1. KKAy mice have a mutation of Ay, which leads to ectopic expression of the agouti coat-color protein, causing a dominantly inherited syndrome of obesity and yellow fur (Siracusa, L. D., Trends Genet. 10: 423-428, 1994). The agouti protein acts as an antagonist for the melanocortine receptors, which are considered to act as a repressor receptor of appetite in the central nervous system (Friedman, J. M. Nature 404: 632-634, 2000; Siracusa, L. D., Trends Genet. 10: 423-428, 1994). KKAy males were bred with ERRL1 females, and their offsprings were selected by hair color (yellow, KKAy(+); black, KKAy(-)) and ERRL1 transgene expression. Comparison of the body weights of each group revealed that the ERRL1 transgene significantly suppressed the body weight increases of KKAy(+) mice (Figs. 6A and 6B), demonstrating that increased ERRL1 expression can antagonize obesity caused by a genetic abnormality.

### 3. Discussion

From the above results, it was confirmed that ERRL1 functions as a 'protein ligand' for ERRs and controls energy expenditure in vivo. In other words, this indicates that by increasing the expression level of ERRL1 in, for example skeletal muscle, this functions as a ERR protein ligand this time, and activates MCAD gene expression to lead to activation of fatty acid β-oxidation. When fatty acid β-oxidation is increased, as a result, accumulated fat will be decreased. It is predicted that the same effects can be achieved by activating the transcriptional ability of ERR and by increasing MCAD gene expression. These effects are very similar to a physiological state which is achieved by physical exercises.

In addition, the above results clearly indicate that the body weight can be decreased by pharmacological activation of ERRL1 or ERR while maintaining the usual calorie intake or more.

### Industrial Applicability

As described in detail above, according to the invention of this application, there are provided a method of screening an ingredient in a remedy for obesity or diabetes by using the interaction between a nuclear orphan receptor ERR and its ligand molecule ERRL1 as an index and a novel drug containing a substance specified by this method as the active ingredient.

## Claims

1. A method of screening a substance which serves as the active ingredient in a drug for obesity and/or diabetes, which comprises treating cells or an animal with a candidate substance and specifying the candidate substance fulfilling one or more of the following requirements as the target active ingredient substance:
(a) increasing the expression level of a ligand factor ERRL1 for a nuclear receptor ERR;
(b) increasing the transcriptional activity of the nuclear receptor ERR;
(c) promoting the binding of ERRL1 to ERR; and
(d) increasing the expression level of an MCAD gene product.

2. A drug for obesity and/or diabetes comprising as the active ingredient one or more substances specified by the method of claim 1.

3. A transgenic non-human animal having a purified polynucleotide encoding a ligand factor ERRL1 for a nuclear receptor ERR in its genomic DNA and overexpressing the ligand factor ERRL1.
